# EUROPEAN PATENT SPECIFICATION

(11) **EP 3 698 769 B1**
(45) Date of publication and mention of the grant of the patent: **07.06.2023**
(21) Application number: 19158835.9
(22) Date of filing: 22.02.2019
(51) Int. Cl.: A61K 8/21, A61K 8/36, A61K 8/73, A61Q 11/00

(54) **AN ORAL REMINERALIZATION ACCELERATING COMPOSITION**
ORALE REMINERALISIERUNGSBESCHLEUNIGENDE ZUSAMMENSETZUNG
COMPOSITION ACCÉLÉRANT LA REMINÉRALISATION ORALE

(43) Date of publication of application: 26.08.2020
(73) Proprietor: De Mars, Steven, 1785 Merchtem (BE); 247 BVBA, 9420 Erpe-Mere (BE); Devos, Frederic, 1652 Alsemberg (BE)
(72) Inventor: De Mars, Steven, 1785 Merchtem (BE); Devos, Frederic, 1652 Alsemberg (BE); De Pus, Evert, 9420 Erpe-Mere (BE)
(74) Representative: IP HILLS NV

(56) References cited:
- FR-A1- 2 822 700
- US-A1- 2015 265 517
- US-A1- 2015 297 490
- US-A1- 2018 092 817

## Description

### Field of the Invention

The present invention generally relates to dental care. More particularly, the present invention aims at stopping the demineralization process and accelerating the remineralization process, which is the human's natural defense mechanism against acid attacks of teeth that makes use of the human's saliva.

### Background of the Invention

Teeth undergo chemical wear as a result of demineralization of the teeth enamel layer as a consequence of acid attacks. Such acid attacks can be direct attacks as a result of the consumption of sour food, like for instance the drinking of cola, or can be indirect attacks as a result of bacterial working using sugar to produce acid. Whereas direct acid attacks lead to surface demineralization and tooth enamel erosion, indirect acid attacks lead to hypodermic demineralization and caries.

The human's saliva neutralizes the pH of tooth plaque through diffusion. This natural defense mechanism against acid attacks is known as remineralization. Stephan's curve shows for indirect acid attacks how much time the remineralization phase takes (time needed for the human's saliva to bring the tooth plaque pH above a critical pH level of tooth structure, typically equal to 5,5 for enamel and 6,5 for dentin) after a given demineralization phase by acid producing bacteria. Stephen's curve shows that demineralization typically takes 30 to 45 minutes and depends on the quality and quantity of the human's saliva. The human teeth typically can stand 3 to 4 acid attacks per day. The curve for direct acid attacks is different. The pH drop for direct acid attacks is often lower. The duration of the remineralization phase depends on the buffer capacity and the titratable acidity of the direct acid.

Conventional teeth brushing corresponding to 2 à 3 times per day, preferably supplemented with teeth flossing and/or the use of interdental brushes is well known for dental care, and therefore recommended by dentists. Teeth brushing removes tooth plaque. Since tooth plaque or biofilm serves as a protective layer against direct acid attacks, erosion of the tooth structure through direct acid attacks increases as a result of frequent teeth brushing.

Another known method of dental care relies on the use of the Cariex mouth spray whose recommended dose is 3 sprays per day. The Cariex mouth spray comes in small bottles of 15 ml and contains amongst others sodium bicarbonate and xylitol. The Cariex mouth spray is advertised at the URLs:
https://www.amazon.com/BRUX-Srl-Cariex-Dental-Spray/dp/B00VXECF7I and
https://www.ncbi.nlm.nih.gov/pubmed/27813150
   and described in European patent EP2666517B1 as an anti-caries product comprising sodium bicarbonate, xylitol and a mucoadhesive polymer.

Sodium bicarbonate has the effect of increasing the pH, hence accelerating the remineralization after an acid attack. Xylitol has the effect of saturating bacteria such that they no longer use sugar to produce acid, hence reducing indirect acid attacks as a result of sweet food consumption. However, in order to have the effect of saturating bacteria such that they no longer use sugar to produce acid, a total daily dose of 3 to 8 grams of xylitol is required for a clinical effect. Dosing frequency should be a minimum of 2 times a day, not to exceed 8 grams per day. This minimum volume is not achieved by 3 sprays of Cariex. Cariex further does not reduce the critical pH of the tooth structure.

United States Patent Application US 2015/0297490 A1 entitled "Compositions, Uses and Methods for Treating or Preventing Dental Caries" describes in [0054] a first example composition for inhibiting dental caries in which 30 weight percent of xylitol, 0,5 weight percent of sodium bicarbonate and 0,1 weight percent of sodium alginate are present.

United States Patent Application US 2018/0092817 A1 entitled "Methods and Compositions for Preventing and Treating Tooth Erosion" describes an oral composition comprising xylitol and crosslinked polyvinylpyrrolidone, abbreviated PVP in a ratio of about 5:1. The oral composition is in the form of a lozenge or lollipop. Embodiments of the oral composition have alkalinizing agents neutralizing acids by raising the pH level of acidic saliva. According to paragraphs [0038]-[0040] of US 2018/0092817 A1, the alkalinizing agent can be selected from at least 15 possible agents and may be present in concentrations that range from 1 % to 8 %. Paragraphs [0041]-[0042] of US 2018/0092817 A1 further specify that the alkalinizing agents may comprise a hardening agent re-hardening softened tooth enamel. Also the rehardening agents can be selected from another list of 10 possible substances and may be present in concentrations ranging from 0,01 % to 10 %. Embodiments of the oral composition also have remineralizing agents for preventing tooth erosion through replacement of mineral components that have been depleted from the teeth. According to paragraphs [0046]-[0048], the remineralizing agents can be selected from about 15 substances and may be present in concentrations ranging from 0,01 % to 10 %. As is further specified in paragraph [0064] of US 2018/0092817 A1, embodiments of the oral composition may comprise xylitol in an amount ranging from 20 % to 95 %. As an alternative to lozenges or lollipops, the oral composition may be in the form of a tooth gel, tooth paste, mouth rinse or mouth wash, as mentioned in paragraph [0052] of US 2018/0092817 A1.

US 2018/0092817 A1 leaves the reader with the difficulty of selecting how many alkalinizing agents, how many remineralizing agents, which alkalinizing agents, and which remineralizing agents will be combined in an effective oral composition. US 2018/0092817 A1 further leaves the reader with the difficulty to select concentrations for the different alkalinizing agents and remineralizing agents within the given intervals once the selection of these agents is made. As a result, US 2018/0092817 A1 brings the risk of producing an oral composition with for instance an overdose of fluorine that may cause fluorisis. Also the described doses of xylitol in US 2018/0092817 A1 contravene international norms. Xylitol has many side effects such as diarrhea and at least the described dose to make 10 lollipops in US 2018/0092817 A1 increases the risk for such side effects. Despite the overdose of some compounds, the oral composition of US 2018/0092817 A1 enables to raise the user's saliva pH to about 7 or greater in about 1 minute, which remains a fairly slow neutralization of the human's saliva pH.

It is consequently an object of the present invention to disclose an oral composition that accelerates the remineralization process faster, with a reduced risk for side effects. It is a further object to disclose such oral composition that accelerates pH neutralization, supports the function of saliva, reduces tooth erosion, reduces caries, reduces gum infection and sensitive teeth while being easy and comfortable in use.

### Summary of the Invention

According to the present invention, one or more of the above-identified drawbacks of the existing solutions are resolved by the oral remineralization accelerating composition defined by claim 1, comprising:
- a first pH increasing component wherein the first pH increasing component is selected to be sodium bicarbonate, having a concentration in the range from 1 to 10 weight/volume percent, preferably in the subrange from 2 to 5 weight/volume percent;
- sodium alginate, having a concentration in the range from 0,5 to 5 weight/volume percent;
- a bacteria saturating component wherein the bacteria saturating component is selected to be xylitol, having a concentration in the range from 10 to 80 weight/volume percent, preferably in the range from 20 to 40 weight/volume percent; and
- a critical pH decreasing component, wherein the critical pH decreasing component is selected from the group of casein phosphopeptide and sodium fluoride, having a preferred concentration of 0,05 weight/volume percent when the critical pH decreasing component is selected to be sodium fluoride, or having a preferred concentration of 2 weight/volume percent when the critical pH decreasing component is selected to be casein phosphopeptide, such that the critical pH of enamel is reduced to a value below 5,5.

Thus, the invention resides in an oral remineralization accelerator that comprises at least four active components, and that is typically applied 1 à 3 times per day. A first active substance is a pH increasing component, sodium bicarbonate. Sodium bicarbonate has an immediate or short-term effect of increasing the pH of acidic saliva, hence accelerating the remineralization of teeth after an acid attack. Sodium bicarbonate is found to preferably have a concentration in the range from 1 to 10 weight/volume percent, further preferably in the subrange from 2 to 5 weight/volume percent to optimize the effect of increasing the pH. A second active substance is sodium alginate. Sodium alginate has the effect of softening tooth plaque hence enabling to remove tooth plaque easier through brushing. Sodium alginate further has the effect of forming raft structures that further neutralize the pH of acidic saliva. Sodium alginate is found to preferably have a concentration between 0,5 and 5 weight/volume percent. A third active substance is xylitol. Xylitol has the effect of reducing the drop in pH, the so-called demineralization, in case of an indirect acid attack through saturating the bacteria that use sugar to produce acid. Xylitol has a long-term effect. It must be used in appropriate doses during a few weeks in order to have a satisfying saturating effect for bacteria. Xylitol is found to preferably have a concentration between 10 and 80 weight/volume percent, further preferably in the subrange from 20 to 40 weight/volume percent. The minimum volume of 6 grams of xylitol per day to be effective hence is achieved through applying 10 millilitres of mouth gel according to the invention, 2 times per day, assuming a xylitol weight/volume percent equal to 30. 10 millilitres of mouth gel could be considered to constitute a single normal dose, applied by a spoon and evenly distributed over the teeth by the human's tongue. Two normal doses could for instance be extended with a third dose of 5 millilitres daily for persons with a higher caries risk. This way, the total daily dose of xylitol still does not exceed 8 grams. Alternatively, persons with a higher caries risk may use 3 normal doses of 10 ml of mouth gel comprising a lower xylitol weight/volume percentage, for example 25 weight/volume percent xylitol. Persons with a low caries risk could be advised to use a single dose of 10 millilitres of mouth gel daily. The risk for side-effects of xylitol like diarrhea by doses exceeding 8 grams of xylitol per day, are minimized this way. A fourth active substance is a critical pH decreasing component, either casein phosphopeptide (CPP) or sodium fluoride. CPP or sodium fluoride has the effect of hardening softened tooth enamel hence improving the resistance of teeth against an acid attack. In appropriate doses, CPP or sodium fluoride for instance reduces the critical pH from 5,5 to 5,0 thus reducing the time required to bring the pH above the critical pH after an acid attack. CPP or sodium fluoride is found to preferably have a concentration between 0,02 and 5 weight/volume percent in the oral remineralization accelerator according to the invention.

The oral remineralization accelerator according to the invention accelerates pH neutralization after a direct or indirect acid attack of teeth, supports the function of saliva for instance in case of low quantity or low quality of the saliva, and reduces the recommended teeth brushing frequency and therefore also the drawbacks of teeth brushing. The oral remineralization accelerator consequently reduces caries resulting from indirect acid attacks and reduces tooth erosion resulting from direct acid attacks. The oral remineralization accelerator in addition reduces gums infections and sensitive teeth.

In case the remineralization accelerating composition is in the form of a mouth gel, the preferred concentration of sodium fluoride is 0,05 weight/volume percent.

In case the remineralization accelerating composition is in the form of a mouth gel, the preferred concentration of PPH is 2 weight/volume percent.

In an embodiment of the oral remineralization accelerating composition according to the invention, as defined by claim 2, the first pH increasing component is selected to be sodium bicarbonate, having a preferred concentration of 2,5 weight/volume percent.

In case the remineralization accelerating composition is in the form of a mouth gel, the preferred concentration of sodium bicarbonate indeed is 2,5 weight/volume percent.

In an embodiment of the oral remineralization accelerating composition according to the invention, as defined by claim 3, the first pH increasing component is selected to be sodium bicarbonate, having a preferred concentration of 4 weight/volume percent.

In case the remineralization accelerating composition is in the form of a mouth rinsing fluid, the preferred concentration of sodium bicarbonate is 4 weight/volume percent.

In an embodiment of the oral remineralization accelerating composition according to the invention, as defined by claim 4, sodium alginate has a preferred concentration of 1 weight/volume percent.

In an embodiment of the oral remineralization accelerating composition according to the invention, as defined by claim 5, xylitol has a preferred concentration of 30 weight/volume percent.

In case the remineralization accelerating composition is in the form of a mouth gel or in the form of a mouth rinsing fluid, the preferred concentration of xylitol indeed is 30 weight/volume percent. This enables to achieve the minimal effective dose of 6 grams per day by recommending application of the oral remineralization accelerating composition 2 or 3 times per day, while avoiding exceeding the dose of 8 grams per day above which side-effects of xylitol may occur.

In an embodiment of the oral remineralization accelerating composition according to the invention, as defined by claim 6, the critical pH decreasing component is selected to be sodium fluoride, having a preferred concentration of 0,0225 weight/volume percent.

In case the remineralization accelerating composition is in the form of a mouth rinsing fluid, the preferred concentration of sodium fluoride is 0,0225 weight/volume percent.

Embodiments of the oral remineralization accelerating composition according to the invention, as defined by claim 7, further comprise:
- a second pH increasing component wherein the second pH increasing component is selected to be calcium carbonate, having a concentration in the range from 2 to 5 weight/volume percent.

Thus, embodiments of the oral remineralization accelerating composition comprise both sodium bicarbonate and calcium carbonate. Calcium carbonate also has the effect of increasing the pH of acidic saliva but to a smaller extent than sodium bicarbonate. Calcium carbonate further gives a chalky aspect to the oral remineralization accelerator in the form of a mouth gel. This chalky aspect is not used in embodiments of the oral remineralization accelerator in the form of a rinsing aid as it dimply deposits. The presence of two pH increasing substances allows to increase the pH of acidic saliva even faster, accelerating pH neutralization and reducing tooth erosion, caries, gums infection and sensitive teeth. Sodium bicarbonate and calcium carbonate are found to preferably have a concentration in the range from 2 to 5 weight/volume percent to optimize the effect of increasing the pH.

In an embodiment of the oral remineralization accelerating composition according to the invention, as defined by claim 8, the second pH increasing component is selected to be calcium carbonate, having a preferred concentration of 3 weight/volume percent.

In case the remineralization accelerating composition is in the form of a mouth gel, the preferred concentration of calcium carbonate indeed is 3 weight/volume percent.

An embodiment of the oral remineralization accelerating composition according to the invention, as defined by claim 9, further comprises:
- menthol having a concentration in the range from 0,2 to 1 weight/volume percent.

The presence of menthol stimulates the secretion of saliva and a fresh feeling or minty taste in the mouth throughout application of the remineralization accelerating composition without the addition of limonene. Alternatively, the remineralization accelerating composition could be given a fruity taste through additives not comprising limonene to avoid allergic side-effects, and not comprising other substances listed on the INCI-list for having allergic effects.

In an embodiment of the oral remineralization accelerating composition according to the invention, as defined by claim 10, menthol has a preferred concentration of 0,5 weight/volume percent.

In case the remineralization accelerating composition is in the form of a mouth gel, the preferred concentration of menthol indeed is 0,5 weight/volume percent for a normal mint taste. As soon as the concentration of menthol exceeds 1,0 weight/volume percent, a strong mint taste is achieved.

In an embodiment of the oral remineralization accelerating composition according to the invention, as defined by claim 11, menthol has a preferred concentration of 0,3 weight/volume percent.

In case the remineralization accelerating composition is in the form of a mouth rinsing fluid, the preferred concentration of menthol is 0,3 weight/volume percent for a mild mint taste.

An embodiment of the oral remineralization accelerating composition according to the invention, as defined by claim 12, further comprises:
- a tooth adhesive.

The presence of a tooth adhesive brings the advantage that a longer working of the oral remineralization accelerating composition is guaranteed.

An embodiment of the oral remineralization accelerating composition according to the invention, as defined by claim 13, has the form of a mouth gel.

A mouth gel brings the advantage it is convenient to apply. It doesn't require a tooth brush, the presence of water, etc., as a result of which it can easily be applied at work, at school, etc.

An embodiment of the oral remineralization accelerating composition according to the invention, as defined by claim 14, has the form of a mouth rinsing solution.

### Brief Description of the Drawings

Fig. 1A and Fig. 1B respectively illustrate the process of dental erosion as a result of direct acid attacks and the process of dental decay as a result of indirect acid attacks;
Fig. 2 shows Stephan's curve in case of an indirect acid attack;
Fig. 3 shows the demineralization phase and remineralization phase in Stephan's curve, as well as the critical pH for tooth enamel;
Fig. 4 shows the effect of sodium bicarbonate, a first component of the oral remineralization accelerating composition according to the invention, on Stephan's curve; the effect may be enhanced in embodiments comprising calcium carbonate;
Fig. 5 shows the effect of sodium alginate, a second component of the oral remineralization accelerating composition according to the invention, on Stephan's curve;
Fig. 6 shows the effect of xylitol, a third component of the oral remineralization accelerating composition according to the invention, on Stephan's curve;
Fig. 7 shows the effect of casein phosphopeptide or sodium fluoride, a fourth component of the oral remineralization accelerating composition according to the invention, on Stephan's curve; and
Fig. 8 shows the combined effect of the oral remineralization accelerating composition according to the invention, on Stephan's curve.

### Detailed Description of Embodiment(s)

A first embodiment of the oral remineralization accelerating composition according to the invention consists of a mouth gel with following constitution:
- purified water with a concentration of 19,50 weight/volume percent;
- magnesium aluminium silicate with a concentration of 1,0 weight/volume percent;
- sodium bicarbonate with a concentration of 2,5 weight/volume percent;
- calcium carbonate with a concentration of 3,0 weight/volume percent;
- sorbitol with a concentration of 24,57 weight/volume percent;
- glycerin with a concentration of 12,442 weight/volume percent;
- sodium alginate with a concentration of 1,0 weight/volume percent;
- xylitol with a concentration of 30,0 weight/volume percent;
- PEG-40 hydrogenated castar oil / propylene glycol with a concentration of 3,5 weight/volume percent;
- menthol with a concentration of 0,5 weight/volume percent;
- carprylyl glycol with a concentration of 0,5 weight/volume percent;
- sodium fluoride with a concentration of 0,05 weight/volume percent;
- citric acid 50 % solution in water with a concentration of 0,058 weight/volume percent; and
- sodium phosphate with a concentration of 1,38 weight/volume percent.

A second embodiment of the oral remineralization accelerating composition according to the invention consists of a mouth gel with following constitution:
- purified water with a concentration of 19,50 weight/volume percent;
- magnesium aluminium silicate with a concentration of 1,0 weight/volume percent;
- sodium bicarbonate with a concentration of 2,5 weight/volume percent;
- calcium carbonate with a concentration of 3,0 weight/volume percent;
- sorbitol with a concentration of 22,62 weight/volume percent;
- glycerin with a concentration of 12,442 weight/volume percent;
- sodium alginate with a concentration of 1,0 weight/volume percent;
- xylitol with a concentration of 30,0 weight/volume percent;
- PEG-40 hydrogenated castar oil / propylene glycol with a concentration of 3,5 weight/volume percent;
- menthol with a concentration of 0,5 weight/volume percent;
- carprylyl glycol with a concentration of 0,5 weight/volume percent;
- casein phosphopeptide or CPP with a concentration of 2,0 weight/volume percent;
- citric acid 50 % solution in water with a concentration of 0,058 weight/volume percent; and
- sodium phosphate with a concentration of 1,38 weight/volume percent.

A third embodiment of the oral remineralization accelerating composition according to the invention consists of a mouth gel with following constitution:
- purified water with a concentration of 57,012 weight/volume percent;
- sodium alginate with a concentration of 1,0 weight/volume percent;
- sodium bicarbonate with a concentration of 2,5 weight/volume percent;
- calcium carbonate with a concentration of 3,0 weight/volume percent;
- xylitol with a concentration of 30,0 weight/volume percent;
- PEG-40 hydrogenated castar oil / propylene glycol with a concentration of 4,0 weight/volume percent;
- menthol with a concentration of 0,5 weight/volume percent;
- carprylyl glycol with a concentration of 0,5 weight/volume percent;
- sodium fluoride with a concentration of 0,05 weight/volume percent;
- citric acid 50 % solution in water with a concentration of 0,058 weight/volume percent; and
- sodium phosphate with a concentration of 1,38 weight/volume percent.

A fourth embodiment of the oral remineralization accelerating composition according to the invention consists of a mouth rinsing fluid with following constitution:
- purified water with a concentration of 52,638 weight/volume percent;
- ethyl alcohol with a concentration of 10,0 weight/volume percent;
- menthol with a concentration of 0,3 weight/volume percent;
- PEG-40 hydrogenated castar oil / propylene glycol with a concentration of 1,5 weight/volume percent;
- sodium alginate with a concentration of 1,0 weight/volume percent;
- sodium anisate with a concentration of 0,5 weight/volume percent;
- sodium saccharin with a concentration of 0,03 weight/volume percent;
- sodium fluoride with a concentration of 0,0225 weight/volume percent;
- sodium bicarbonate with a concentration of 4,0 weight/volume percent;
- xylitol with a concentration of 30,0 weight/volume percent; and
- CI 42090 aquatic solution with a concentration of 0,01 weight/volume percent.

It is noticed that in the above described embodiments, the weight/volume percentage of sodium alginate may be increased to levels above 1 weight/volume percent, in replacement of an equivalent weight/volume percentage of purified water. The weight/volume percentage of sodium alginate may also be decreased to levels below 1 weight/volume percent and be replaced by an equivalent weight/volume percentage of xanthan gum.

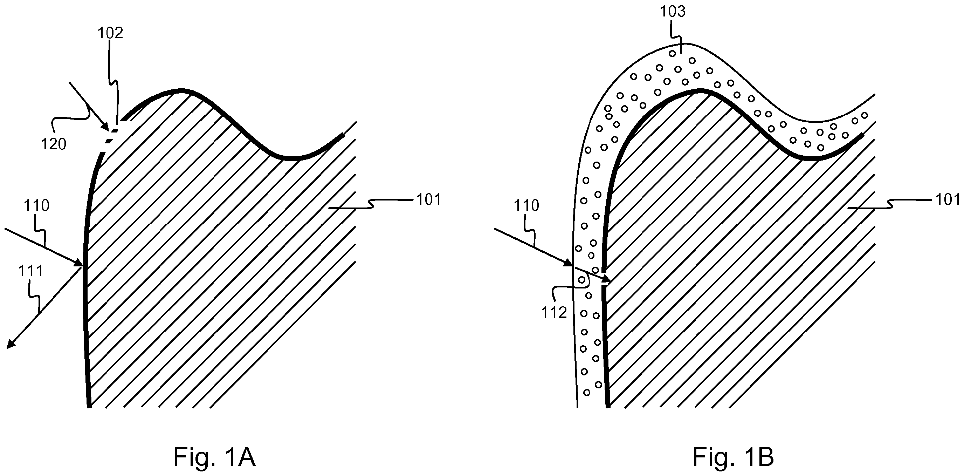

Fig. 1A illustrates dental decay as a result of a direct acid attack 120 on a tooth whose tooth plaque has been removed through recent brushing. The tooth shown in Fig. 1A undergoes chemical decay as a result of demineralization 102 of the tooth enamel 101 as a consequence of the direct acid attack 120. Such direct acid attack 120 results from the consumption of sour food like for instance the drinking of cola. Sugars 110 are less detrimental for brushed teeth as they are washed away without affecting the tooth enamel 101, as is indicated by arrow 111 in Fig. 1A.

Fig. 1B shows an unbrushed tooth with enamel layer 101 and tooth plaque 103. Tooth plaque 103 protects the enamel layer 101 against direct acid attacks like the one illustrated by Fig. 1A but it stimulates indirect acid attacks that result from sweet food consumption. Bacteria present in tooth plaque 103 use sugars 110 to produce acid 112 that has a demineralizing effect on tooth enamel 101, leading to caries. Whereas frequent tooth brushing to remove tooth plaque 103 reduces the risk for indirect acid attacks, erosion of tooth enamel through direct acid attacks increases as a result of frequent teeth brushing because tooth plaque 103 serves as a protective layer against direct acid attacks.

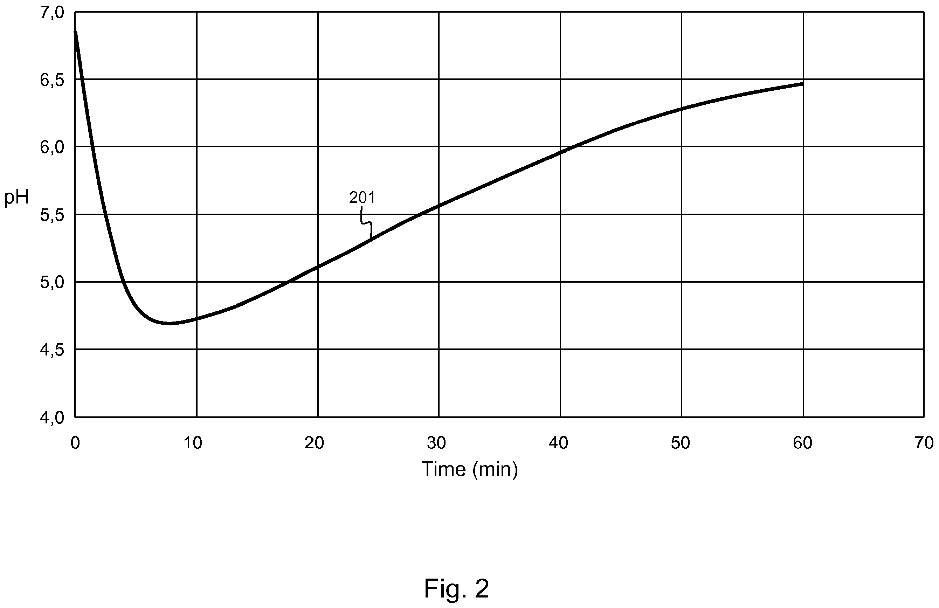

The evolution over time of the pH of tooth plaque 103 after an acid attack is reflected in Stephan's curve 201, shown in Fig. 2. After such acid attack, the pH drops to levels below 5,0 within 5 to 10 minutes. Thereafter, the pH slowly increases again. As Stephan's curve 201 shows, it typically takes 30 minutes to 45 minutes to bring the tooth plaque pH above the critical pH level of tooth structure, which is 5,5 for tooth enamel and 6,5 for dentin.

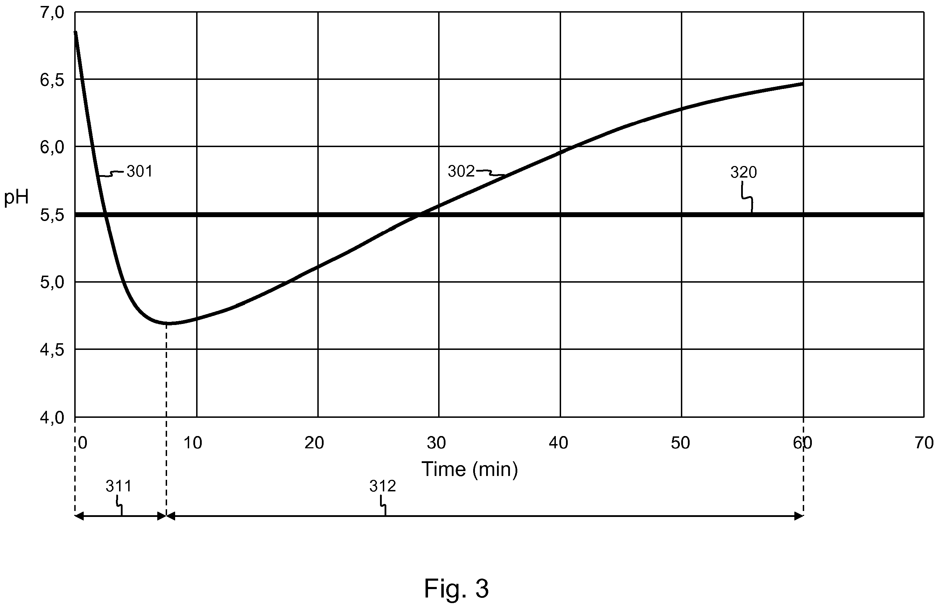

The human's saliva neutralizes the pH of tooth plaque 103 through diffusion. This natural defence mechanism against acid attacks is known as remineralization. As is illustrated by Fig. 3, Stephan's curve consequently shows a demineralization phase 301, being a short period 311 of 5 to 10 minutes wherein an acid attack results in a fast drop of the pH of tooth plaque, followed by a remineralization phase 302, being a relatively long period 312 of 30 to 45 minutes wherein the human's saliva again neutralizes the pH of tooth plaque. The length in time of the demineralization phase 302 depends on the quality and quantity of the human's saliva. Generally, it takes about 30 minutes to raise the pH of tooth plaque above the critical pH level 320 which is 5,5 for tooth enamel.

Fig. 3 shows the demineralization phase and remineralization phase in Stephan's curve, as well as the critical pH;

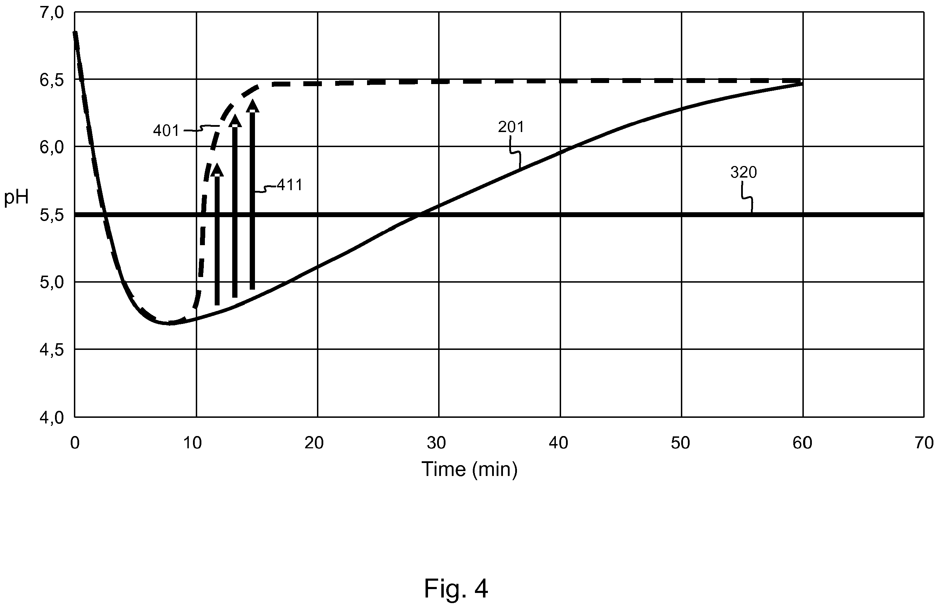

Fig. 4 shows the effect of sodium bicarbonate, a first active component of the oral remineralization accelerating composition according to the invention, on Stephan's curve. The effect may be enhanced in embodiments comprising calcium carbonate as additional active component. Sodium bicarbonate and calcium carbonate have the effect of increasing the pH of tooth plaque after an acid attack. The effect is immediate or short-term. As is indicated by arrows 411, sodium bicarbonate and possibly calcium carbonate turn Stephan's curve 201 into a new curve 401 with accelerated remineralization phase.

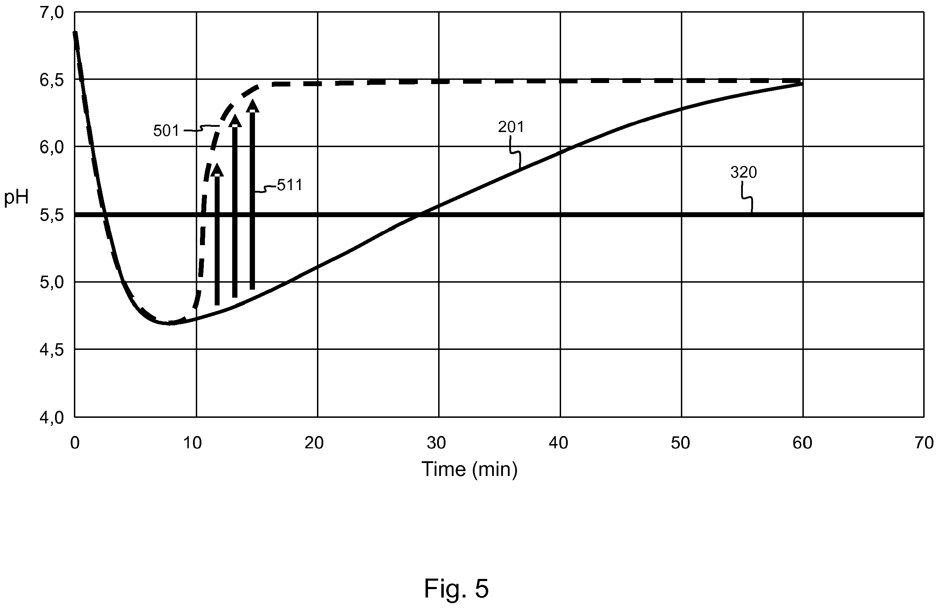

Fig. 5 shows the effect of sodium alginate, a second active component of the oral remineralization accelerating composition according to the invention, on Stephan's curve. Sodium alginate has the effect of forming raft structures that neutralize the pH of acidic saliva. Just like sodium bicarbonate or calcium carbonate, sodium alginate consequently has the effect of increasing the pH of tooth plaque after an acid attack. As is indicated by arrows 511, sodium alginate turns Stephan's curve 201 into a new curve 501 with accelerated remineralization phase. Sodium alginate further has the effect of softening tooth plaque, enabling to remove tooth plaque easier through brushing.

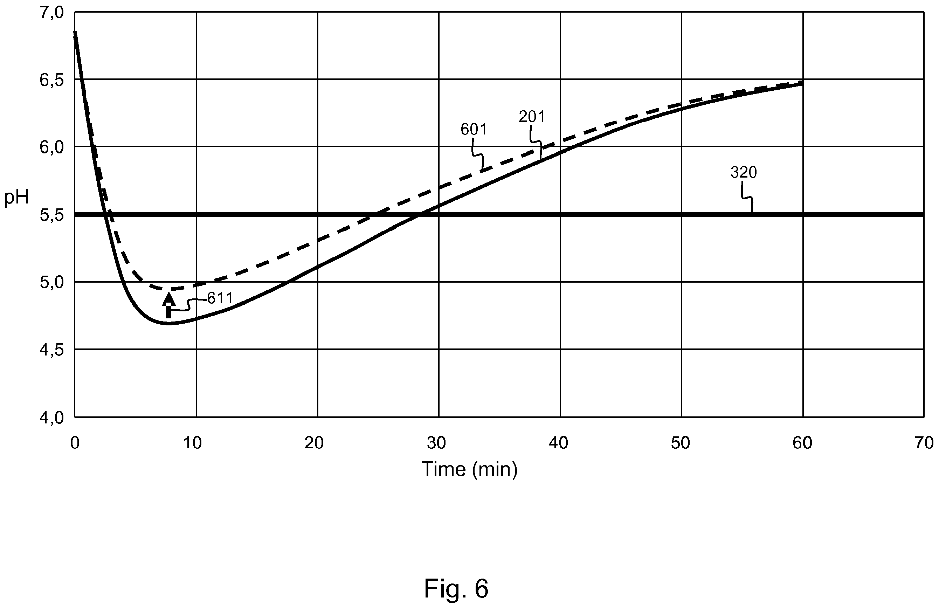

Fig. 6 shows the effect of xylitol, a third active component of the oral remineralization accelerating composition according to the invention, on Stephan's curve. Xylitol is a bacteria saturating component having the effect of reducing the drop in pH or so-called demineralization in case of an acid attack. This is indicated by arrow 611 in Fig. 6. As a result of the presence of xylitol, Stephan's curve 201 is turned into a curve 601 with higher pH minimum resulting from an indirect acid attack. The effect of xylitol is a long-term effect. Xylitol must be used in appropriate doses during a few weeks for an optimal bacteria saturating effect.

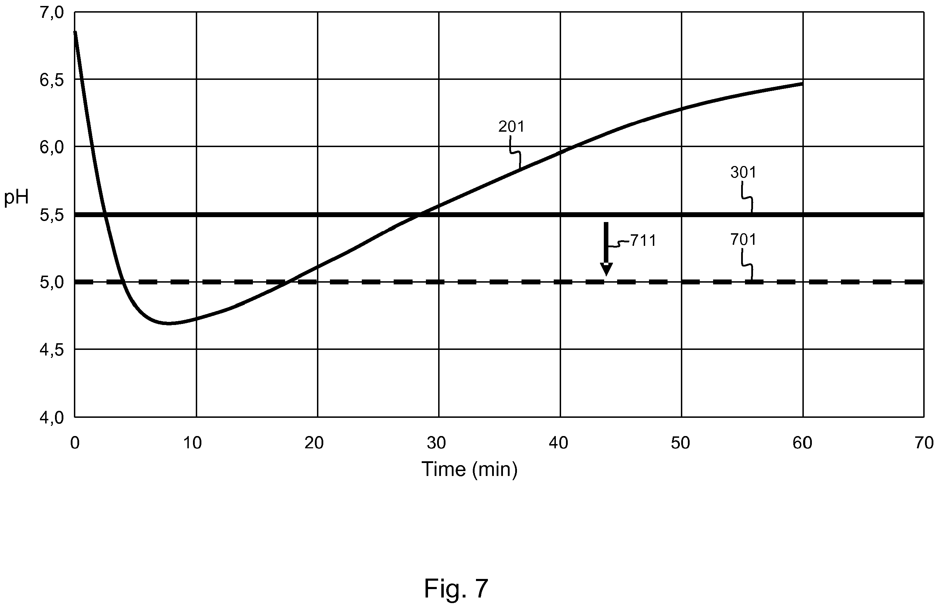

Fig. 7 shows the effect of casein phosphopeptide or sodium fluoride, a fourth active component of the oral remineralization accelerating composition according to the invention, on Stephan's curve. Casein phosphopeptide or sodium fluoride improves the resistance of teeth against an acid attack. In other words, CPP or sodium fluoride decreases the critical pH of tooth enamel, for instance from 5,5 to 5,0 as is indicated by arrow 711 in Fig. 7, where the new critical pH is denoted by 701. As a consequence of the lowered critical pH 701, the time required to bring the pH of tooth plaque above the critical pH after an acid attack is reduced.

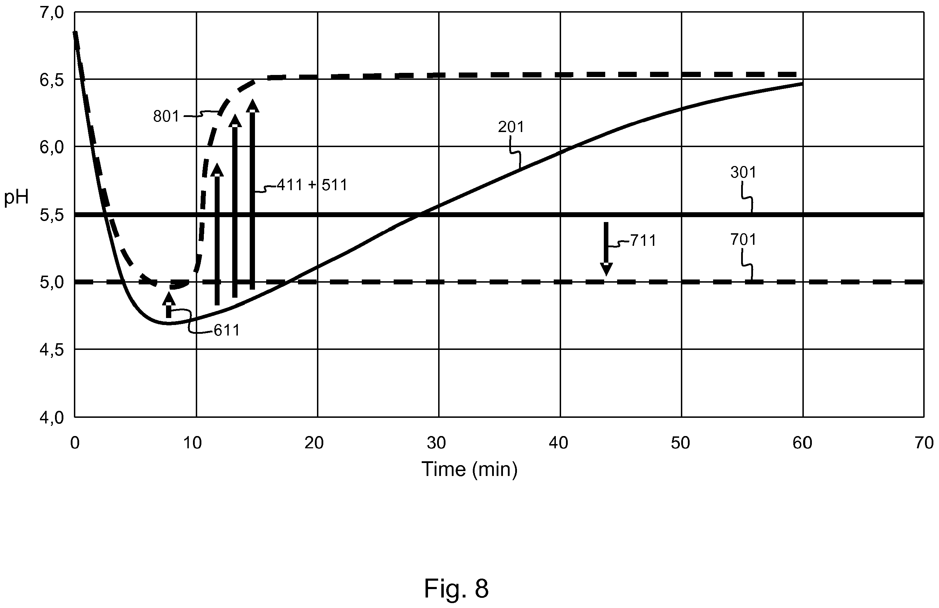

Fig. 8 shows the combined effect of the oral remineralization accelerating composition according to the invention, on Stephan's curve 201. The pH increasing effect 411 of sodium bicarbonate and possibly calcium carbonate, the pH neutralizing effect 511 of sodium alginate, the bacteria saturating effect 611 of xylitol, and the critical pH reducing effect 711 of CPP or sodium fluoride have the combined effect of turning Stephan's curve 201 into a new curve 801 that does not drop below the critical pH of tooth plaque after an acid attack, or that stays only for a very limited time of a few seconds up to a few minutes below the critical pH of tooth plaque after an acid attack.

It is noticed that a possible synergistic effect of the combined presence of both CPP and sodium fluoride in embodiments of the oral remineralization accelerating composition according to the invention requires further clinical research.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. It is to be understood that the terms so used are interchangeable under appropriate circumstances and embodiments of the invention are capable of operating according to the present invention in other sequences, or in orientations different from the one(s) described or illustrated above.

## Claims

1. An oral remineralization accelerating composition comprising:
- a first pH increasing component wherein the first pH increasing component is selected to be sodium bicarbonate, having a concentration in the range from 1 to 10 weight/volume percent, preferably in the subrange from 2 to 5 weight/volume percent;
- sodium alginate, having a concentration in the range from 0,5 to 5,0 weight/volume percent;
- a bacteria saturating component wherein the bacteria saturating component is selected to be xylitol, having a concentration in the range from 10 to 80 weight/volume percent, preferably in the subrange from 20 to 40 weight/volume percent; and
- a critical pH decreasing component, wherein the critical pH decreasing component is selected from the group of casein phosphopeptide and sodium fluoride, having a preferred concentration of 0,05 weight/volume percent when said critical pH decreasing component is selected to be sodium fluoride, or having a preferred concentration of 2 weight/volume percent when said critical pH decreasing component is selected to be casein phosphopeptide, such that the critical pH of enamel is reduced to a value below 5,5.

2. An oral remineralization accelerating composition according to claim 1, wherein the first pH increasing component is selected to be sodium bicarbonate, having a preferred concentration of 2,5 weight/volume percent.

3. An oral remineralization accelerating composition according to claim 1, wherein the first pH increasing component is selected to be sodium bicarbonate, having a preferred concentration of 4 weight/volume percent.

4. An oral remineralization accelerating composition according to one of the preceding claims, wherein sodium alginate has a preferred concentration of 1 weight/volume percent.

5. An oral remineralization accelerating composition according to one of the preceding claims, wherein xylitol has a preferred concentration of 30 weight/volume percent.

6. An oral remineralization accelerating composition according to one of the preceding claims, wherein said critical pH decreasing component is selected to be sodium fluoride, having a preferred concentration of 0,0225 weight/volume percent.

7. An oral remineralization accelerating composition according to one of the preceding claims, further comprising:
- a second pH increasing component wherein the second pH increasing component is selected to be calcium carbonate, having a concentration in the range from 2 to 5 weight/volume percent.

8. An oral remineralization accelerating composition according to claim 7, wherein the second pH increasing component is selected to be calcium carbonate, having a preferred concentration of 3 weight/volume percent.

9. An oral remineralization accelerating composition according to one of the preceding claims, further comprising:
- menthol having a concentration in the range from 0,2 to 1 weight/volume percent.

10. An oral remineralization accelerating composition according to claim 9, wherein menthol has a preferred concentration of 0,5 weight/volume percent.

11. An oral remineralization accelerating composition according to claim 9, wherein menthol has a preferred concentration of 0,3 weight/volume percent.

12. An oral remineralization accelerating composition according to one of the preceding claims, further comprising:
- a tooth adhesive.

13. An oral remineralization accelerating composition according to one of the preceding claims, wherein said oral remineralization accelerating composition has the form of a mouth gel.

14. An oral remineralization accelerating composition according to one of the preceding claims, wherein said oral remineralization accelerating composition has the form of a mouth rinsing solution.

## Patentansprüche

1. Zusammensetzung zum Beschleunigen der oralen Remineralisierung, umfassend:
- eine erste pH-erhöhende Komponente, wobei die erste pH-erhöhende Komponente ausgewählt ist als Natriumbicarbonat mit einer Konzentration im Bereich von 1 bis 10 Gewichts-/Volumenprozent, vorzugsweise im Unterbereich von 2 bis 5 Gewichts-/Volumenprozent;
- Natriumalginat mit einer Konzentration im Bereich von 0,5 bis 5,0 Gewichts-/Volumenprozent,
- eine bakteriensättigende Komponente, wobei die bakteriensättigende Komponente ausgewählt ist als Xylit mit einer Konzentration im Bereich von 10 bis 80 Gewichts-/Volumenprozent, vorzugsweise im Unterbereich von 20 bis 40 Gewichts-/Volumenprozent; und
- eine den kritischen pH-Wert senkende Komponente, wobei die den kritischen pH-Wert senkende Komponente ausgewählt ist aus der Gruppe aus Casein-Phosphopeptid und Natriumfluorid mit einer bevorzugten Konzentration von 0,05 Gewichts-/Volumenprozent, wenn die den kritischen pH-Wert senkende Komponente als Natriumfluorid ausgewählt ist, oder mit einer bevorzugten Konzentration von 2 Gewichts-/Volumenprozent, wenn die den kritischen pH-Wert senkende Komponente als Casein-Phosphopeptid ausgewählt ist, sodass der kritische pH-Wert des Zahnschmelzes auf einen Wert unter 5,5 reduziert wird.

2. Zusammensetzung zum Beschleunigen der oralen Remineralisierung gemäß Anspruch 1, wobei die erste pH-erhöhende Komponente ausgewählt ist als Natriumbicarbonat mit einer bevorzugten Konzentration von 2,5 Gewichts-/Volumenprozent.

3. Zusammensetzung zum Beschleunigen der oralen Remineralisierung gemäß Anspruch 1, wobei die erste pH-erhöhende Komponente ausgewählt ist als Natriumbicarbonat mit einer bevorzugten Konzentration von 4 Gewichts-/Volumenprozent.

4. Zusammensetzung zum Beschleunigen der oralen Remineralisierung gemäß einem der vorhergehenden Ansprüche, wobei Natriumalginat eine bevorzugte Konzentration von 1 Gewichts-/Volumenprozent aufweist.

5. Zusammensetzung zum Beschleunigen der oralen Remineralisierung gemäß einem der vorhergehenden Ansprüche, wobei Xylit eine bevorzugte Konzentration von 30 Gewichts-/Volumenprozent aufweist.

6. Zusammensetzung zum Beschleunigen der oralen Remineralisierung gemäß einem der vorhergehenden Ansprüche, wobei die den kritischen pH-Wert senkende Komponente ausgewählt ist als Natriumfluorid mit einer bevorzugten Konzentration von 0,0225 Gewichts-/Volumenprozent.

7. Zusammensetzung zum Beschleunigen der oralen Remineralisierung gemäß einem der vorhergehenden Ansprüche, ferner umfassend:
- eine zweite pH-erhöhende Komponente, wobei die zweite pH-erhöhende Komponente ausgewählt ist als Calciumcarbonat mit einer Konzentration im Bereich von 2 bis 5 Gewichts-/Volumenprozent.

8. Zusammensetzung zum Beschleunigen der oralen Remineralisierung gemäß Anspruch 7, wobei die zweite pH-erhöhende Komponente ausgewählt ist als Calciumcarbonat mit einer bevorzugten Konzentration von 3 Gewichts-/Volumenprozent.

9. Zusammensetzung zum Beschleunigen der oralen Remineralisierung gemäß einem der vorhergehenden Ansprüche, ferner umfassend:
- Menthol mit einer Konzentration im Bereich von 0,2 bis 1 Gewichts-/Volumenprozent.

10. Zusammensetzung zum Beschleunigen der oralen Remineralisierung gemäß Anspruch 9, wobei Menthol eine bevorzugte Konzentration von 0,5 Gewichts-/Volumenprozent aufweist.

11. Zusammensetzung zum Beschleunigen der oralen Remineralisierung gemäß Anspruch 9, wobei Menthol eine bevorzugte Konzentration von 0,3 Gewichts-/Volumenprozent aufweist.

12. Zusammensetzung zum Beschleunigen der oralen Remineralisierung gemäß einem der vorhergehenden Ansprüche, ferner umfassend:
- einen Zahnklebstoff.

13. Zusammensetzung zum Beschleunigen der oralen Remineralisierung gemäß einem der vorhergehenden Ansprüche, wobei die Zusammensetzung zum Beschleunigen der oralen Remineralisierung die Form eines Mundgels aufweist.

14. Zusammensetzung zum Beschleunigen der oralen Remineralisierung gemäß einem der vorhergehenden Ansprüche, wobei die Zusammensetzung zum Beschleunigen der oralen Remineralisierung die Form einer Mundspüllösung aufweist.

## Revendications

1. Composition accélérant la reminéralisation orale comprenant :
- un premier composant d'augmentation du pH, ledit premier composant d'augmentation du pH étant choisi pour être du bicarbonate de sodium, présentant une concentration dans la plage de 1 à 10 pour cent en poids/volume, de préférence dans la sous-plage de 2 à 5 pour cent en poids/volume ;
- l'alginate de sodium, présentant une concentration dans la plage de 0,5 à 5,0 pour cent en poids/volume ;
- un composant de saturation de bactéries, ledit composant de saturation de bactéries étant choisi pour être le xylitol, présentant une concentration dans la plage de 10 à 80 pour cent en poids/volume, de préférence dans la sous-plage de 20 à 40 pour cent en poids/volume ; et
- un composant de diminution du pH critique, ledit composant de diminution du pH critique étant choisi dans le groupe du phosphopeptide de caséine et du fluorure de sodium, présentant une concentration préférée de 0,05 pour cent en poids/volume lorsque ledit composant de diminution du pH critique est choisi pour être le fluorure de sodium, ou présentant une concentration préférée de 2 pour cent en poids/volume lorsque ledit composant de diminution du pH critique est choisi pour être le phosphopeptide de caséine, de sorte que le pH critique de l'émail soit réduit à une valeur inférieure à 5,5.

2. Composition accélérant la reminéralisation orale selon la revendication 1, ledit premier composant d'augmentation du pH étant choisi pour être du bicarbonate de sodium, présentant une concentration préférée de 2,5 pour cent en poids/volume.

3. Composition accélérant la reminéralisation orale selon la revendication 1, ledit premier composant d'augmentation du pH étant choisi pour être du bicarbonate de sodium, présentant une concentration préférée de 4 pour cent en poids/volume.

4. Composition accélérant la reminéralisation orale selon l'une des revendications précédentes, ledit alginate de sodium présentant une concentration préférée de 1 pour cent en poids/volume.

5. Composition accélérant la reminéralisation orale selon l'une des revendications précédentes, ledit xylitol présentant une concentration préférée de 30 pour cent en poids/volume.

6. Composition accélérant la reminéralisation orale selon l'une des revendications précédentes, ledit composant de diminution du pH critique étant choisi pour être le fluorure de sodium, présentant une concentration préférée de 0,0225 pour cent en poids/volume.

7. Composition accélérant la reminéralisation orale selon l'une des revendications précédentes, comprenant en outre :
- un second composant d'augmentation du pH, ledit second composant d'augmentation du pH étant choisi pour être du carbonate de calcium, présentant une concentration dans la plage de 2 à 5 pour cent en poids/volume.

8. Composition accélérant la reminéralisation orale selon la revendication 7, ledit second composant d'augmentation du pH étant choisi pour être du carbonate de calcium, présentant une concentration préférée de 3 pour cent en poids/volume.

9. Composition accélérant la reminéralisation orale selon l'une des revendications précédentes, comprenant en outre :
- du menthol présentant une concentration dans la plage de 0,2 à 1 pour cent en poids/volume.

10. Composition accélérant la reminéralisation orale selon la revendication 9, ledit menthol présentant une concentration préférée de 0,5 pour cent en poids/volume.

11. Composition accélérant la reminéralisation orale selon la revendication 9, ledit menthol présentant une concentration préférée de 0,3 pour cent en poids/volume.

12. Composition accélérant la reminéralisation orale selon l'une des revendications précédentes, comprenant en outre :
- un adhésif dentaire.

13. Composition accélérant la reminéralisation orale selon l'une des revendications précédentes, ladite composition accélérant la reminéralisation orale ayant la forme d'un gel buccal.

14. Composition accélérant la reminéralisation orale selon l'une des revendications précédentes, ladite composition accélérant la reminéralisation orale ayant la forme d'une solution de rinçage buccal.
